# EUROPEAN PATENT SPECIFICATION

(11) **EP 1 214 904 B1**
(45) Date of publication and mention of the grant of the patent: **20.02.2008**
(21) Application number: 01129635.7
(22) Date of filing: 12.12.2001
(51) Int. Cl.: A61B 5/024, A61B 5/022

(54) **Wrist-worn vital sign detection device**
Am Handgelenk getragene Biosignaldetektionsvorrichtung
Dispositif de détection de signaux vitaux se portant au poignet

(30) Priority: 13.12.2000 JP 2000378437; 21.06.2001 JP 2001188252
(43) Date of publication of application: 19.06.2002
(73) Proprietor: Omron Healthcare Co., Ltd., Kyoto 615-0084 (JP)
(72) Inventor: Itonaga, Kazunobu, c/o Omron Corporation, Shiokoji-dori,Shimogyo-ku, Kyoto-shi, Kyoto 600-8530 (JP); Tanabe, Kazuhisa, c/o Omron Corporation, Shiokoji-dori,Shimogyo-ku, Kyoto-shi, Kyoto 600-8530 (JP); Ohtani, Toshio, c/o Omron Corporation, Shiokoji-dori,Shimogyo-ku, Kyoto-shi, Kyoto 600-8530 (JP); Oku, Shojiro, c/o Omron Corporation, Shiokoji-dori,Shimogyo-ku, Kyoto-shi, Kyoto 600-8530 (JP); Tanaka, Takahide, c/o Omron Corporation, Shiokoji-dori,Shimogyo-ku, Kyoto-shi, Kyoto 600-8530 (JP); Sano, Yoshihiko, c/o Omron Corporation, Shiokoji-dori,Shimogyo-ku, Kyoto-shi, Kyoto 600-8530 (JP); Kato, Hiroyuki, c/o Omron Corporation, Shiokoji-dori,Shimogyo-ku, Kyoto-shi, Kyoto 600-8530 (JP)
(74) Representative: Kilian, Helmut

(56) References cited:
- EP-A- 0 756 849
- EP-A- 1 048 267
- WO-A-00/62666
- WO-A-97/12542
- US-A- 4 993 422
- US-A- 5 652 955
- US-A- 6 132 383
- US-A- 6 142 966
- PATENT ABSTRACTS OF JAPAN vol. 1999, no. 05, 31 May 1999 (1999-05-31) & JP 11 033007 A (NIPPON COLIN CO LTD), 9 February 1999 (1999-02-09)

## Description

### BACKGROUND OF THE INVENTION

### FIELD OF THE INVENTION

This invention relates to a vital sign detection device configured to be mounted on a wrist of a subject for vital sign detection, especially to a wrist pulse wave detection device and a wrist blood pressure monitor.

### DESCRIPTION OF THE RELATED ART

Vital sign detection, which is performed on a wrist of a subject of the detection, is known to be susceptible to hand movement. This applies to both measuring the pulse wave itself and measuring the blood pressure through the detection of the pulse wave. For example, the detection of a pulse wave of the artery at the wrist is performed by pressing a pressure sensing unit on the palm side of the wrist. If the movement of the hand is not restricted during detection period, the joint of the wrist is free to move, resulting in a noise generation. Furthermore, the pressure applied through the sensing unit on the wrist varies depending on the angle between the hand and the arm. Variation in that pressure results in an unexpected change in the amplitude of the measured pulse wave. One of the proposed solutions to this problem is to use a supporting member for restricting the movement of hand, which is mounted on the wrist portion separatel y and prior to the mounting of the main body of the pulse wave detection device having the sensing unit (JP 11-033 007 A). However, mounting both the main body and the supporting member separately makes preparation for pulse wave detection bothersome, and requires a complicated structural design of a whole detection unit.

On the other hand, a pressure sensing unit of a wrist blood pressure monitor, which is provided between an air bag and a deflation valve, detects air pressure value and a pulse wave combined in the varying air pressure during deflation of compressed air from the air bag. If a hand used for blood pressure detection is significantly bent toward its palm side, the tendon at the wrist portion protrudes near the skin, pushing the artery deep under the skin. This results in insufficient pressurizing of the detection portion and, thus, an inaccurate detection. It is noted, however, that pulse wave detection requires more rigorous restriction of the movement of hand so as not to generate noises, while blood pressure detection needs only to restrict the inward movement of the hand so as not to hinder the detection.

A vital sign detection device comprising a regulating portion for restricting the movement of the hand, which is not adjustable is also shown in WO-A-97/12542.

This document forms the basis for the preamble of claims 1 and 5.

### SUMMARY OF THE INVENTION

The invention is directed to a vital sign detec tion device which has a simple structure for easy mounting and yet provides vital signs with a high accuracy by restricting the movement of hand.

The invention provides a vital sign detection device as defined in claims 1 and 5.

In one embodiment in which the invention provides a pulse wave detection device, the regulating portion is a plate extending from the main body on the palm side . The device may have plates on both sides of the hand. A band may be attached to one of the plates for further restricting the movement of the hand. There may be a hole in the band for inserting a finger. When mounting the device on a wrist, a person can hold onto the band by inserting a finger into the hole while adjusting the position of the device without any help from others. A positioning mark may be formed on the plate or the band for an accurate positioning of the fingers. Since the tendon on which the main body should be placed is aligned with the middle finger, an accurate positioning of the finger results in an accurate positioning of the main body.

According to the invention as defined in claim 1, the regulating portion is the main body itself or a part of the main body, which is moved by a moving mechanism toward the distal portion of the hand. This movement of the main body may activate the device for operation. This configuration eliminates a need for an operation-activation switch from the main body, resulting in a larger top surface area of the main body available for other purposes including information display. The regulating portion may also be a guide portion formed as a part of the main body, which can be extended toward the distal end of the hand for further restricting the movement of the hand. The guide portion may be detached from the main body.

According to the invention as defined in claim 5, the vital sign detection device is configured to be mounted on a wrist of a subject having a main body, a pressure sensing unit for sensing vital sign, a pressurizing unit for pressing the pressure sensing unit on a detection portion of the wrist, a cuff configured for mounting the main body on the palm side of the wrist and a regulating portion for restricting movement of the hand. This regulating portion extends along the direction of the hand, is configured to be placed on the palm side of the hand, and comprises two parts integral to each other, which are adjustable so that the length of the regulating portion is adjusted according to the size of the hand. The regulating portion may be provided on either side of the hand, or on both sides. A band may be attached to one or both of the regulating portions.

### BRIEF DESCRIPTION OF THE DRAWINGS

FIG. 1 is a perspective view of a pulse wave detection device of a first configuration.
FIG. 2 is a perspective view of a clip portion used with the first embodiment of FIG. 1.
FIG. 3 is a perspective view of a pulse wave detection device of a second configuration.
FIG. 4 is a cross-sectional view of the device of FIG. 3.
FIG. 5 is a schematic view of a pulse wave detection device of the second configuration mounted on a wrist.
FIG. 6 is a schematic view of a pulse wave detection device mounted on a wrist of a third configuration.
FIG. 7 is a schematic view of a pulse wave detection device mounted on a wrist of a fourth configuration.
FIG. 8 is a schematic view of a pulse wave detection device mounted on a wrist of a fifth configuration.
FIG. 9 is a schematic view of a pulse wave detection device mounted on a wrist of a sixth configuration.
FIG. 10 is a schematic view of a pulse wave detection device mounted on a wrist of a seventh configuration.
FIG. 11 is a perspective view of a pulse wave detection device of an eighth configuration.
FIG. 12 is a cross-sectional view of the device of FIG. 11.
FIG. 13 is a schematic view of a pulse wave detection device having components of the device consolidated in a main body.
FIG. 14 is a perspective view of a blood pressure monitor having common elements.
FIG. 15 is a schematic cross-sectional view when the device of FIG. 14 is mounted on a wrist.
FIG. 16 shows a posture when a hand is bent toward its palm side.
FIG. 17 is a schematic cross-sectional view of the wrist portion of the hand bent toward the palm side as shown in FIG. 16.
FIG. 18 is a schematic side view of a blood pressure monitor mounted on a wrist of an embodiment of the invention.
FIG. 19 is a schematic side view of the device of FIG. 18 in which the main body of the device is moved toward the distal end of the hand.
FIG. 20 is a schematic side view of a blood pressure monitor mounted on a wrist of another embodiment of the invention.
FIG. 21 is a schematic plan view of the device of FIG. 20 mounted on the wrist.
FIG. 22 is a schematic plan view of a blood pressure monitor mounted on a wrist as another embodiment of the invention.
FIG. 23 is a schematic plan view of the device of FIG. 22 in which a guide portion is extended toward the distal end of the hand.
FIG. 24 is a schematic side view of a blood pressure monitor mounted on a wrist as a further embodiment of the invention.
FIG. 25 is a schematic side view of the device of FIG. 24 in which a guide portion is rotated out toward the distal end of the hand.
FIG. 26 is a schematic plan view of the device of FIG. 25.

### DETAILED DESCRIPTION OF THE INVENTION

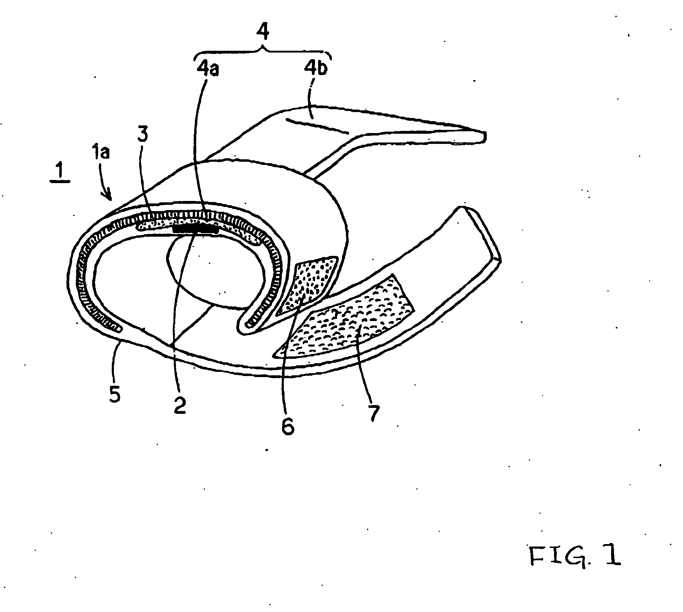

Some embodiments of this invention will be described with reference to the drawings described above. FIG. 1 is a perspective view of a pulse wave detection device 1 including a pressure sensing unit 2, which is in contact with a detection portion of a subject's body (such as the palm side of a wrist) during detection, an air bag 3, which presses the pressure sensing unit 2 on the detection portion, a clip portion 4, which covers the air bag 3 from the outside, and a band 5, which is used as a cuff for mounting the device on the wrist. This band 5 carries or contains the pressure sensing unit 2, the air bag 3, and the clip portion 4.

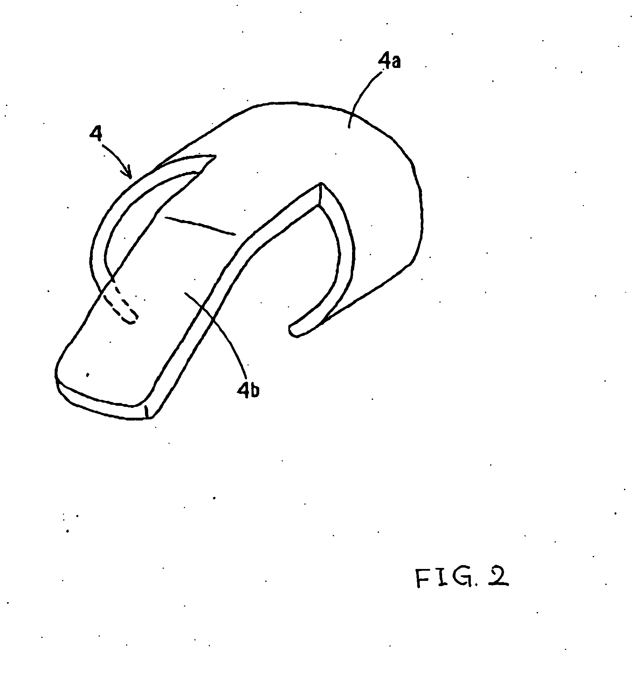

The clip portion 4, as shown in FIG. 2, includes a cylindrical clip base 4a with an opening along its longitudinal direction, and a plate 4b, which extends from one end of the clip base 4a and bends toward the central axis of the cylinder of the cylindrical clip base 4a. The clip base 4a is covered by the band 5, while the plate 4b is exposed without any cover. The portion of the plate 4b next to the clip base 4a is a simple extension of the surface of the cylinder of the clip base 4a, but the top portion of the plate 4b is bent toward the central axis of the cylinder of the clip base 4a. Surface fasteners 6, 7 are mounted on both ends of the band 5 so that the band 5 is fastened when the device is worn on a wrist.

This pulse wave detection device has a main body 1a, which includes the pressure sensing unit 2, air bag 3, clip base 4a and the portion of the band 5 which carries or contains the aforementioned components of the device. The plate 4b extends from the clip base 4a toward the distal end of the hand of the subject wearing the pulse wave detection device 1.

This configuration as well as of the other configurations described below, makes it easier to mount the device on a wrist because of the unitary combination of the main body 1a and the device for restricting the movement of a hand (plate 4b), and yet ensures an accurate and stable detection of pulse wave.

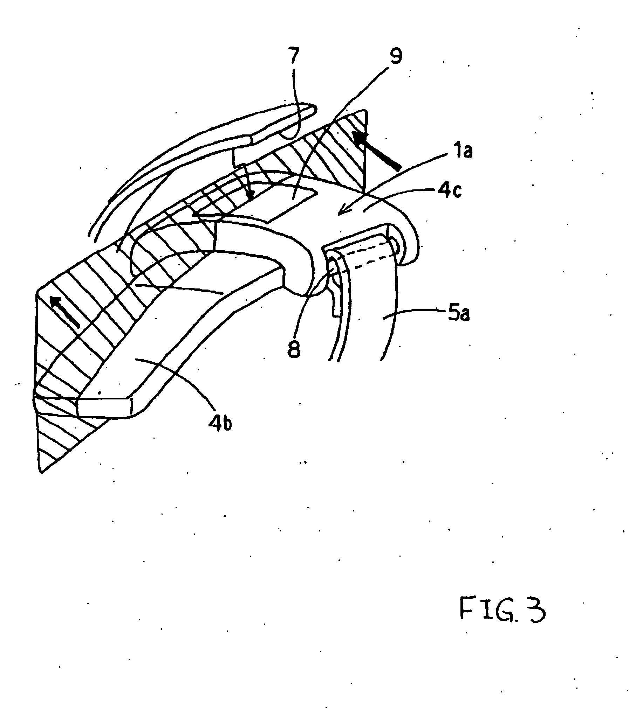

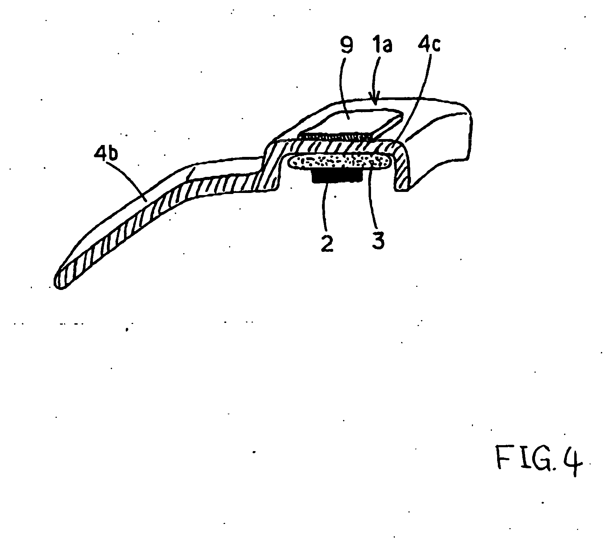

FIG. 3 is a perspective view of a pulse wave detection device and FIG. 4 is a cross-sectional view of the device shown in FIG. 3 through the plane indicated by the arrow in FIG. 3. The portion denoted by reference numeral 4c corresponds to the clip base 4a of the first embodiment shown in FIG. 1 and forms a casing for the main body 1a of the pulse wave detection device. A plate 4b extends from the casing 4c toward the distal end of a hand wearing the pulse wave detection device and bends toward back side of the hand. The size and the shape of the plate 4b are essentially the same as the plate shown in FIGS. 1 and 2. One end of a band 5a is fixed to the casing 4c of the main body 1a through an axis 8 of the casing 4c, and the other end of the band 5a has a surface fastener 7, which engages with a surface fastener 9 mounted on the casing 4c.

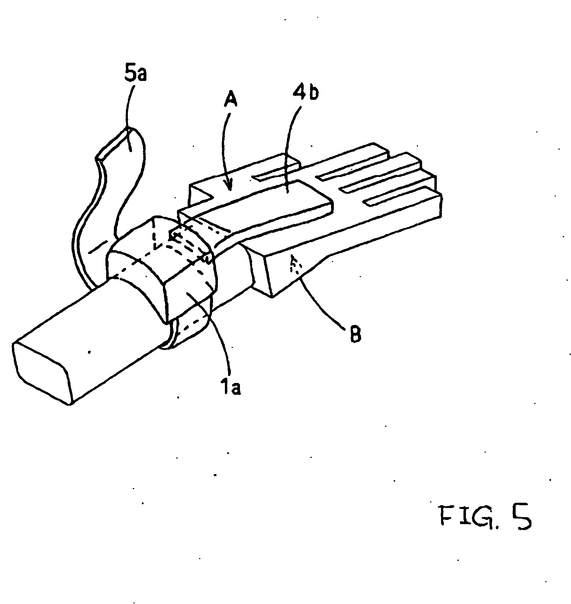

The pulse wave detection device of the second configuration is mounted on a wrist of a subject so that the plate 4b is, as shown in FIG. 5, in direct contact with the palm (A) side of the hand through a proper adjustment of the band 5a and the fasteners 7, 9. The rotation of the wrist joint is restricted during detection, since the main body 1a and the plate 4b hinder the movement of the hand relative to the wrist portion. Similarly, the pulse wave detection device of the first configuration shown in FIG. 1 should be mounted on the wrist of a subject so that the plate 4b is in direct contact with the palm side of the subject's hand.

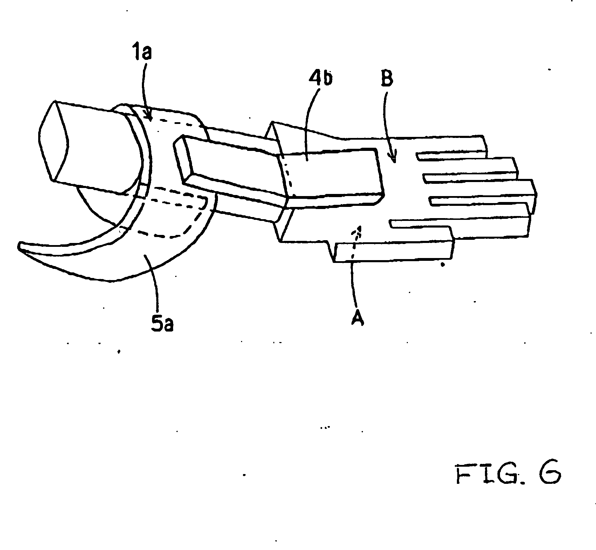

FIG. 6 is a schematic view of a pulse wave detection device of a third configuration. A plate 4b extends from the main body 1a toward the distal end of the hand, and bends toward the back (B) side of the hand. Although it is not shown in FIG. 6, plate 4b is connected to and supported by the main body 1a. The pulse wave detection device of the third configuration is mounted on the wrist so that the plate 4b is in direct contact with the back side of the hand.

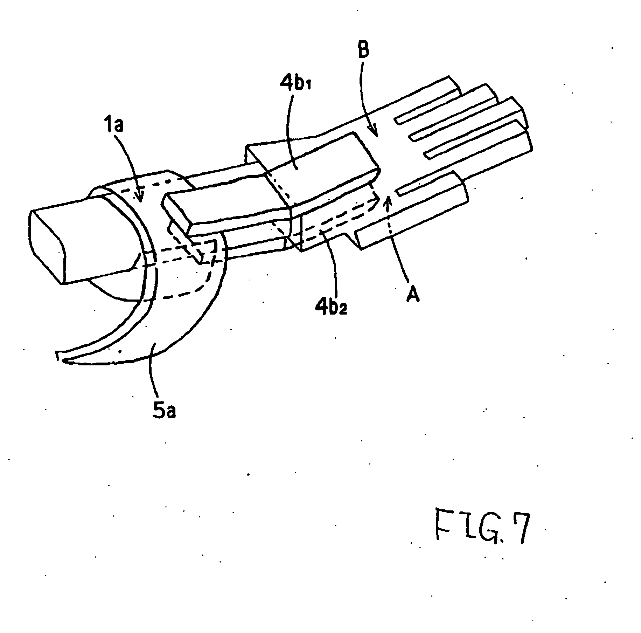

FIG. 7 is a schematic view of a pulse wave detection device of a fourth configuration. This device has two p lates 4b1, 4b2, extending toward the distal end of the hand. Plate 4b1 on the back (B) side of the hand extends from the band 5a, and 4b2 on the palm (A) side extends from the main body 1a. The device is mounted on the wrist so that plate 4b1 is in direct contact with the back side of the hand and plate 4b2 is in direct contact with the palm of the hand. This configuration ensures more stable detection of the pulse wave because the movement of the hand is restricted by the two plates 4b1, 4b2 on both sides of the hand.

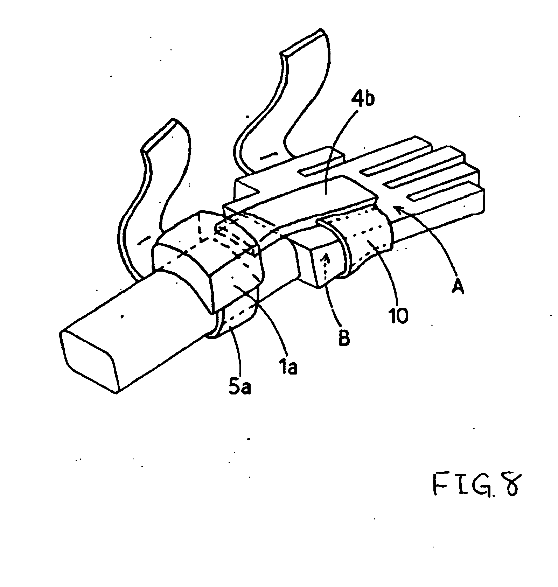

FIG. 8 is a schematic view of a pulse wave detection device of a fifth configuration. This device includes a band 10, which is connected to the plate 4b at its distal end and is used for tying the palm or fingers to the plate 4b, in addition to the band 5a used for mounting the device on the wrist. When a subject wears the device of the fifth configuration on his or her wrist, the subject or another person first temporarily mounts the main body 1a of the pulse wave detection device on the wrist so that the plate 4b is in direct contact with the palm (A) of the hand on which the band 5a is mounted, then adjusts the position of the plate 4b relative to the hand, ties the palm or fingers to the plate 4b using the band 10, and finally tightly mounts the device on the wrist. In this fifth embodiment, the movement of the hand and the rotation of the wrist joint are further restricted because the hand is tied to the plate 4b using the belt 10.

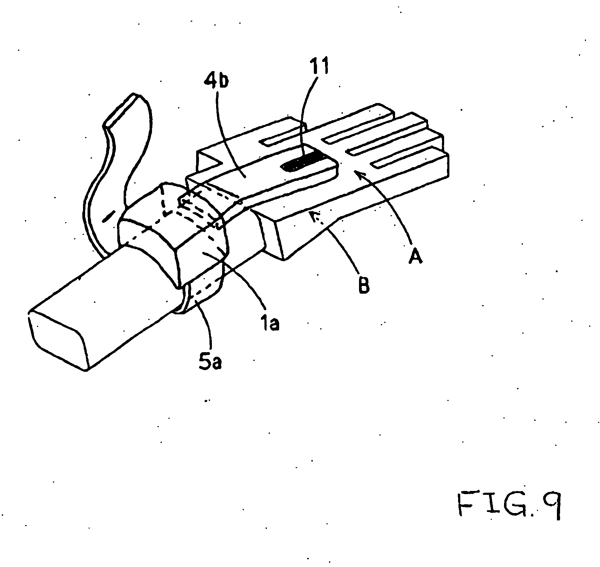

FIG. 9 is a schematic view of a pulse wave detection device of a sixth configuration. This device includes a marking 11 for positioning the fingers, which is formed on the upper surface of the plate 4b at its distal end. The plate 4b extends from the main body 1a and has the marking along the center line of the plate 4b. When the marking 11 coincides with the center line of the middle finger of the hand, as shown in FIG. 9, the pressure sensing unit 2 is placed at or near the artery of the wrist for pulse wave detection. This configuration ensures pulse wave detection of high accuracy by adjusting the mounting of the main body 1a with the band 5a so that the marking 11 lies on the line running through the center of the middle finger.

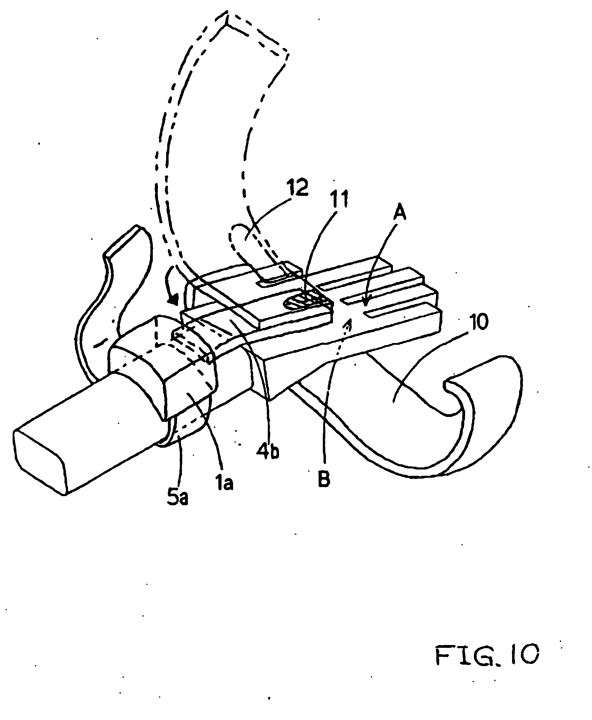

FIG. 10 is a schematic view of a pulse wave detection device of a seventh configuration. As in the case of the fifth embodiment, this device includes a band 10 which is connected to the plate 4b at its distal end and is used for tying the palm or fingers to the plate 4b, in addition to the band 5a used for mounting the device on the wrist. Similarly, the movement of the hand and thus the rotation of the wrist joint are further restricted with the hand being fixed to the plate 4b. In this configuration one end of the band 10 is fixed on the upper surface of the plate 4b at its distal end. A marking 11 is formed for the alignment of the middle finger, and serves the same purpose as the marking 11 of the sixth configuration shown in FIG. 9.

As shown in FIG. 10, the band 10 of the seventh configuration has a hole 12 for receiving the thumb when the band 10 is wrapped around the hand for tying it to the plate 4b. When a subject of detection wears this device, he or she first puts the main body 1a on the wrist so that the plate 4b is in direct contact with the palm (A) of the hand, puts the thumb through the hole 12, grabs the plate 4b with the thumb through the hole 12, and adjusts the alignment between the marking 11 and the middle finger so that a proper positioning of the device relative to the hand is secured by fastening the two bands 5a, 10. This configuration ensures a reliable mounting of the pulse wave detection device even when the subject of detection wears the device by itself.

As one modification of the configurations (1-7) described above, the plate 4b and clip base 4a may be formed as two separate parts and integrally combined through a slide mechanism and fixation by a screw or other fastening structure. In this configuration which falls within the scope of the invention according to claim 5, it is possible to adjust the length of the plate 4b in accordance with the size of the hand of a subject of detection.

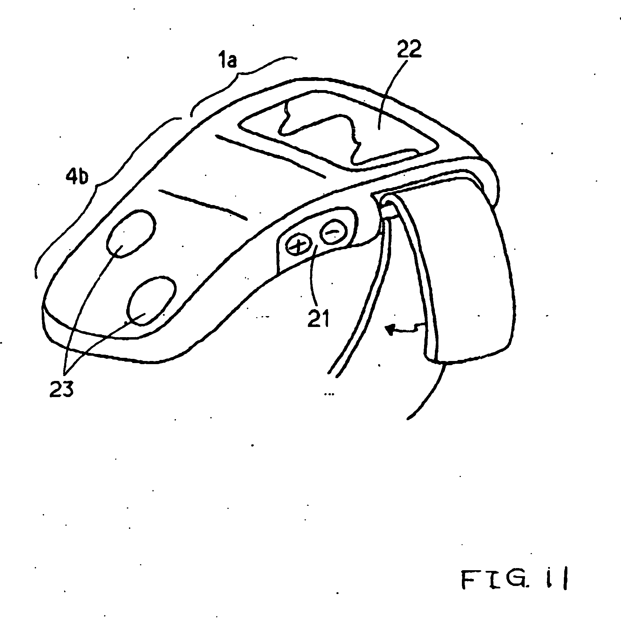

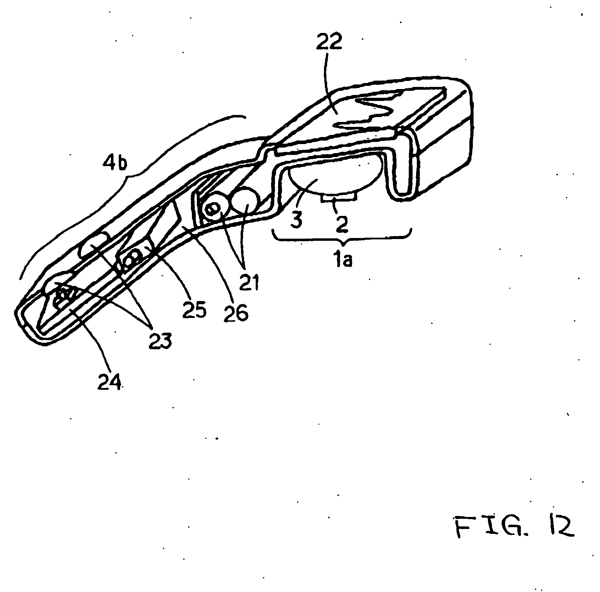

FIG. 11 is a perspective view of a pulse wave detection device of a eighth configuration, and the FIG. 12 is a perspective view of the device shown in FIG. 11 cut along a direction parallel to the longitudinal direction of the device.

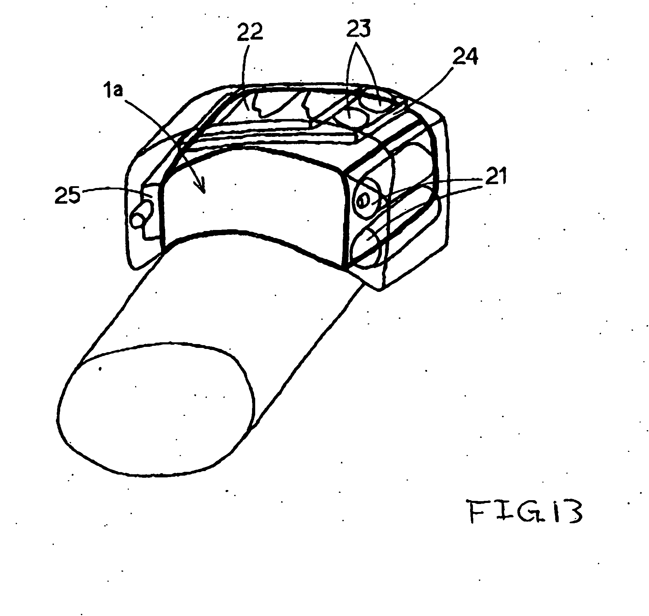

FIG. 13 shows a pulse wave detection device in which batteries 21, a display unit 22, an operation unit 23, a circuit board 24 and a compressed air manipulation system 25 including a pump and a pressure control valve are consolidated within the main body 1a. In this configuration, the size and weight of the main body 1a inevitably increase and thus result in a difficulty of mounting the device on the wrist because of an improper weight balance of the device and a high center of gravity position of the device. The portion indicated by the thick line in FIG. 13 is the core of the device housing only the pressure sensing unit 2 and a pressurizing unit such as an air bag 3.

The eighth configuration shown in FIGS. 11 and 12 is intended to solve the difficulty described in conjunction with the pulse wave detection device shown in FIG. 13. In the eighth configuration, the plate 4b of the clip portion 4 is formed as a casing having a storage area 26 for storing batteries 21, a circuit board 24 and a compressed air manipulation system 25. An operation unit 23 is formed on the top surface of the plate 4b. The plate 4b of this configuration may be the plate 4b of the second configuration or any other configurations described above. A display unit 22 is formed on the top surface of the main body 1, though it may be formed on the top surface of the plate 4b as well.

This configuration reduces the size and weight of the main body 1a by disposing in the plate 4b all or a part of the components of the device described above, including the butteries 21, the display unit 22, the operation unit 23, the circuit board 24 and the compressed air manipulation system 25. As a result, the ease of mounting the device on a wrist improves because of the improved weight balance of the device.

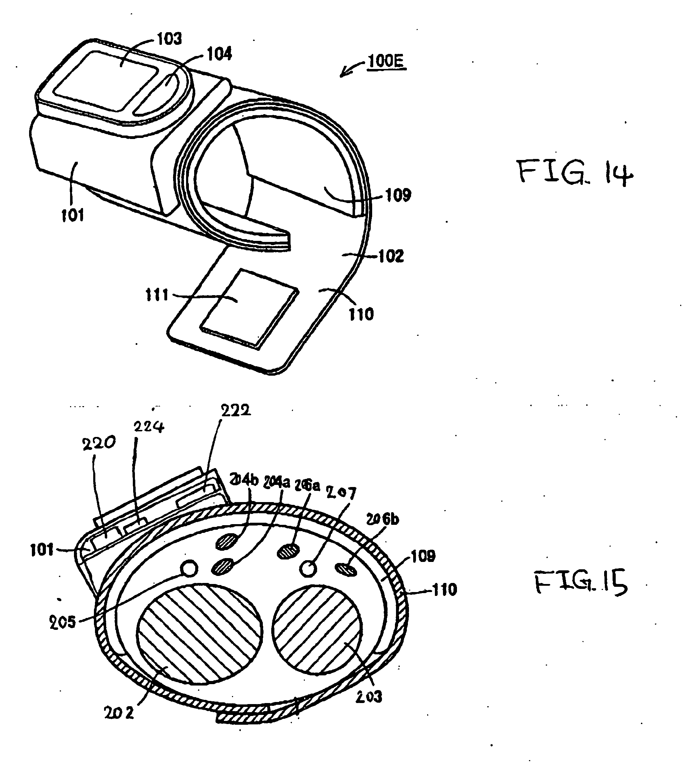

FIG. 14 shows common components of blood pressure monitors described below as embodiments of this invention. A wrist blood pressure monitor 100E includes a main body 101 containing a control device for controlling the blood pressure detection, and a cuff 102 for mounting the main body 101 on a wrist. The main body 101 has a display unit 103 and a start switch 104 on its top surface, and, as shown in FIG. 15, contains a pressure sensing unit 220, a pump 222 for compressing the air, a deflation valve 224 and a CPU for controlling these components of the blood pressure monitor.

The cuff 102 is a band 110 which has an air bag 109 therein for receiving compressed air sent form the pump 222 contained in the main body 101 and for pressurizing the artery of wrist during blood pressure detection. The band also has a surface fastener 111 for fastening the band 110 around the wrist.

In the blood pressure detection, the pressure sensing unit 220 does not directly touch the wrist for detecting the pulse wave of the artery as is the case with the pulse wave detection device described above. Rather, the pressure sensing unit 220 of the blood pressure monitor is placed between the air bag 109 and the deflation valve 224. For measuring blood pressure, the air bag 109 is first filled with compressed air from the pump 222 for pressurizing the wrist portion until the artery is closed, the pumping is then stopped, and the deflation valve 224 is controlled by the CPU for slowly deflating the air bag 109. During the release of the air from the air bag 109, the pressure sensing unit 220 detects the pressure of the air bag 109, which is a combination of the air pressure and pressure due to the pulse wave. The CPU derives the pulse wave component from the total pressure measured, and finds a maximum blood pressure when the pulse wave appears and a minimum blood pressure when the pulse wave disappears. As a modification, it is also possible to find the maximum and minimum blood pressure during the pressurization of the air bag 109 in a converse fashion.

FIG. 15 shows a cross-sectional view of the blood pressure monitor 100E of FIG. 14, which is mounted on a wrist and has the air bag 109 filled with the compressed air for the detection. The main body 101 of the blood pressure monitor 100E contains a pump 222 for generating the compressed air, a deflation valve 224, and a pressure sensing unit 220. The band 110 is fastened so that the air bag 109 is placed over the radialis 205 of the wrist portion for properly pressurizing the radialis 205 during detection.

Also shown in the figure are the radius 202 on the thumb side of the cross section, the ulna 203 on the little finger side, flexor digitorum profundus 204a, palmaris longus 204b, flexor digitorum superficialis 206a, flexor carpi ulnaris 206b, and the ulnaris 207.

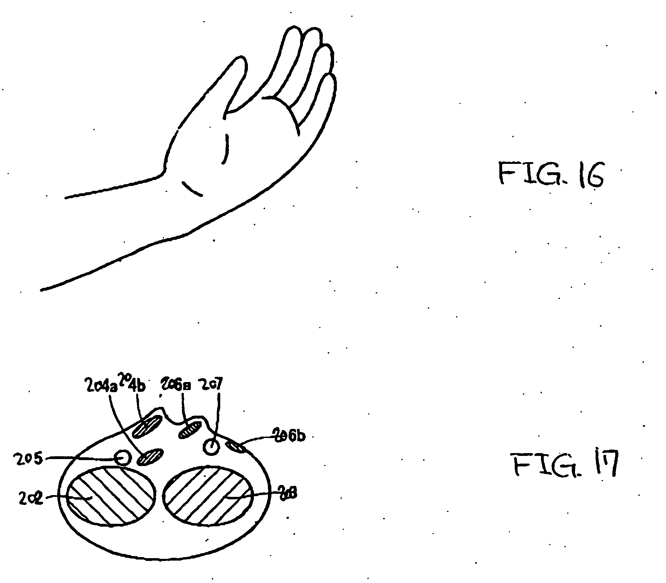

If a hand is bent toward its palm side, as shown in FIG. 16, the tendons of the wrist move toward the skin as shown in FIG. 17. Especially, the palmaris longus 204b and the flexor carpi ulnaris 206b rise in a large amount enough to push the skin upward. On the other hand, the radialis 205 and the ulnaris 207 are pushed down into the muscles. As a result, when a hand is bent toward its palm side during blood pressure detection, it is difficult to properly apply the pressure of the air bag 109 to the radialis 205 or the ulnaris 207 because of the tendons rising toward the skin. Accordingly, accurate detection of blood pressure is not achieved.

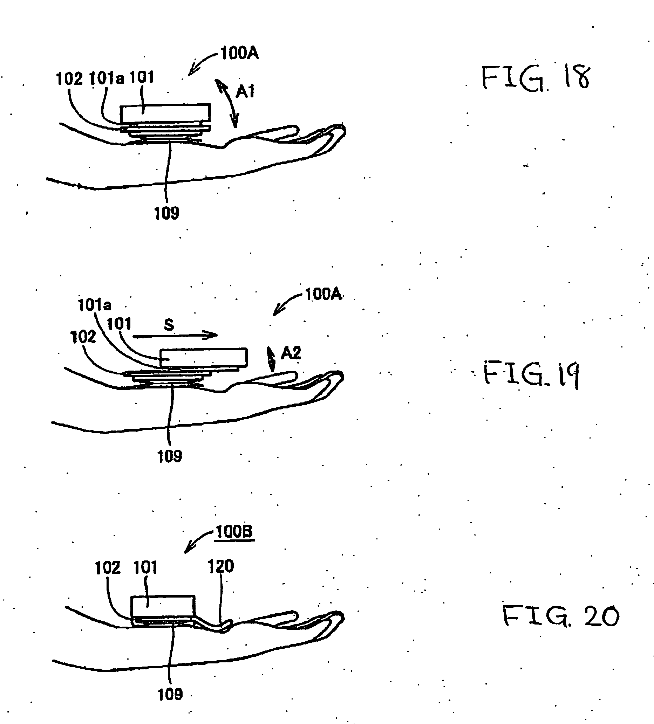

The following embodiments of this invention are directed to solving the aforementioned problem. FIG. 18 is a schematic side view of a blood pressure monitor 100A mounted on a wrist as an embodiment of this invention, and FIG. 19 is a schematic side view of the blood pressure monitor 100A in which the main body of the device is moved toward the distal end of the hand.

The wrist blood pressure monitor 100A of thise embodiment has a main body 101 which is slidably mounted on a cuff 102. The mechanism for moving the main body 101 toward the distal end of the hand (the direction of the arrow S in FIG. 19) is a slide rail 101a provided between the main body 101 and the cuff 102. It is also possible to use other slide mechanism known in the art including linear bearings to replace the slide rail 101a as a means for moving the main body 101 in this embodiment.

The main body 101, when moved toward the distal end of the hand as shown in FIG. 19, serves as a regulating portion for restricting the movement of the hand. The range of motion of the hand is smaller when the main body 101 is moved (A2 in FIG. 19) than it is when the main body 101 is positioned on the cuff (A1 in FIG. 18). As a result, more accurate application of the air bag pressure to the artery is achieved when the main body is moved toward the distal end and thus prevents the hand from bending toward its palm side enough to allow the tendons to hinder blood pressure detection, for example.

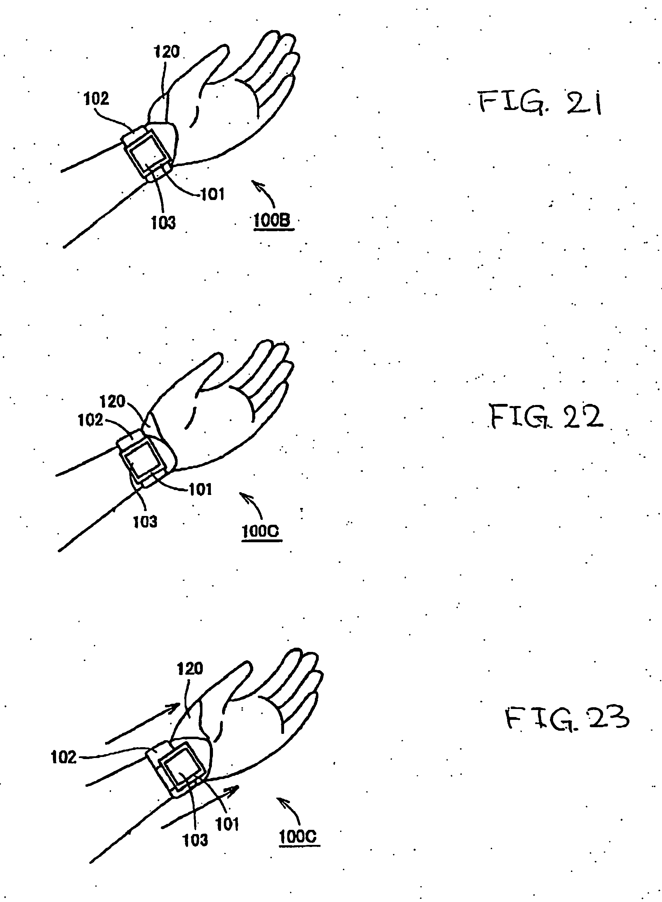

FIG. 20 is a schematic side view of a blood pressure monitor 100B mounted on a wrist as an embodiment of the invention, and FIG. 21 is a schematic plan view of the blood pressure monitor 100B of FIG. 20 mounted on the wrist of a subject.

The wrist blood pressure monitor 100B of this embo diment has a guide portion 120 extending toward the distal end of the hand. This guide portion 120 may be made of a plastic material such as ABS or polypropylene, and may be connected to either the main body 101 or the cuff 102.

The guide portion 120 serves as a regulating portion for restricting the movement of the hand. As a result, more accurate application of the air bag pressure to the artery is achieved because the guide portion 120 prevents the hand from bending toward its palm side enough to allow the tendons to hinder detection. The guide portion 120 may be detachably mounted on the blood pressure monitor 100B, or it may be retractable into the blood pressure monitor 100B itself. Either configuration improves the handling of the blood pressure monitor 100B during its mounting on and detaching from the wrist.

FIG. 22 is a schematic plan view of a blood pressure monitor 100C mounted on a wrist as another embodiment of the invention, and FIG. 23 is a schematic plan view of the device of FIG. 22 in which a guide portion 120 is extended toward the distal end of the hand.

The wrist blood pressure monitor 100C of the embodiment of figure 22 has a guide portion 120 which is the same as the guide portion 120 of the embodiment of figure 20. However, the guide portion 120 of the embodiment of figure 22 is extended toward the distal end of the hand based on a slide mechanism in synchronization with the slide movement of the main body 101 toward the distal end. The slide mechanism of the embodiment of figure 18 may be used in the embodiment of figure 22. The blood pressure monitor 100C also has a switching mechanism which activates the blood pressure monitor 100C when the main body 101 is moved toward the distal end of the hand. The switching mechanism may be based on a pair of a detent and a projection formed between main body 101 and slide mechanism 101a. The engagement of the detent and the projection, for example, switches on the blood pressure monitor 100C, and disengagement of the detent and projection switches off the device. Alternatively, the engagement may switch off the device and the disengagement switch on the device. Other switching mechanisms known in the art may be applied to this embodiment.

The guide portion 120, which extends toward the distal end of the hand in synchronization with the slide movement of the main body 101, serves as a regulating portion for restricting the movement of the hand. As a result, more accurate application of the air bag pressure to the artery is achieved because the guide portion 120 prevents the hand from bending toward its palm side enough to allow the tendons to hinder the detection.

Furthermore, the blood pressure monitor 100C of the eleventh embodiment, which is switched on for the detection only when the main body 101 is moved toward the distal end of the hand, ensures that the regulating portion for restricting the movement of the hand (the main body 101 or the guide portion 120) is always in place during the detection of blood pressure. In addition, this configuration elimina tes the need for a start switch mounted on the top surface of the main body 101, and thus makes it possible to have a larger display unit on the top surface of the main body 101 for better display of the information on the display unit, including measured blood pressure values.

Although the wrist blood pressure monitor of the embodiment of figure 22 has both the slide mechanism for the guide portion 120 in synchronization with the slide movement of the main body 101 and the switching mechanism in synchronizatio n with the same movement, each of the two mechanisms may be provided separately for a blood pressure monitor.

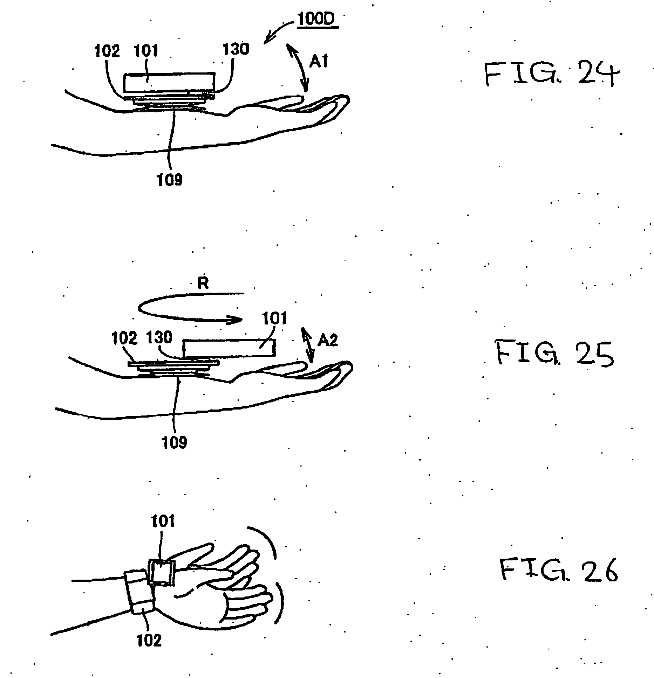

FIG. 24 is a schematic side view of a blood pressure monitor 100D mounted on a wrist as a further embodiment of the invention, and FIG. 25 is a schematic side view of the blood pressure monitor 100D of FIG. 24 in which a guide portion is rotated out toward the distal end of the hand. FIG. 26 is a schematic plan view of the blood pressure monitor 100D of FIG. 25.

The main body 101 of the wrist blood pressure monitor 100D of this embodiment can be rotated out toward the distal end of the hand from the cuff 102. The rotating mechanism of this embodiment is a rotation axis 130 provided between the main body 101 and the cuff 102. As shown in FIG. 25, the main body 101 is swung around the rotation axis 130 in the direction of R shown in the figure.

The main body 101, when rotated out toward the distal end of the hand as shown in FIG. 25, serves as a regulating portion for restricting the movement of the hand. The range of motion of the hand is smaller when the main body 101 is rotated out (A2 in FIG. 25) than it is when the main body 101 is positioned on the cuff (A1 in FIG. 24). As a result, more accurate application of the air bag pressure to the artery is achieved when the main body is moved toward the distal end and prevents the hand from bending toward its palm side enough to allow the tendons to hinder the detection.

The features described above of the different configurations of blood pressure monitors may be selectively combined to provide a wrist blood pressure monitor. Furthermore, although the whole main body is moved toward the distal end in the above embodiments, only a portion of the main body may be moved and a similar effect may be obtained for restricting the movement of the hand.

Although the described configurations are divided into those of a pulse wave detection device and those of a blood pressure monitor, the features described for the pulse wave detection device may be applicable to the blood pressure meter, and those for the blood pressure meter may be applicable to the pulse wave detection device, as long as the features meet the requirements of restricting the movement of hand of a particular device. For example, the plate for restricting the movement of hand of the first described configuration as a pulse wave detection device may be detachably mounted on the main body for the ease of mounting the device on the wrist, or may be extended toward the distal end of the hand for a better restriction of the movement of hand. Likewise, a wrist blood pressure meter may have a plate extending from the main body for properly restricting the movement of hand for an accurate detection.

## Claims

1. A vital sign detection device configured to be mounted on a wrist of a hand of a subject, comprising:
a main body (101) configured to be mounted on a palm side of the wrist;
a pressure sensing unit (220) for sensing a pulse wave;
a pressurizing unit for applying pressure to a detection portion of the wrist;
a cuff (102) configured for mounting the main body on the palm side of the wrist; and
a regulating portion for restricting movement of the hand, said regulating portion being configured to extend along the direction of the hand and being configured to be placed on a palm side of the hand;
**characterized by**
a moving mechanism (101a) for moving the main body (101) or a portion of the main body toward the distal end of the hand so that the main body or the portion of the main body serves as the regulating portion.

2. The vital sign detection device of claim 1, further comprising a switching mechanism which activates the device when the main body (101) or the portion of the main body is moved toward the distal end of the hand.

3. The vital sign detection device of claim 1, wherein the portion of the main body (101) moved toward the distal end is a guide portion (120) extendable toward the distal end.

4. The vital sign detection device of claim 3, wherein the guide portion (120) is detachably mounted on the main body (101).

5. A vital sign detection device configured to be mounted on a wrist of a hand of a subject, comprising:
a main body (1a) configured to be mounted on a palm side of the wrist;
a pressure sensing unit (2) for sensing a pulse wave;
a pressurizing unit for applying pressure to the pressure sensing unit on a detection portion of the wrist;
a cuff (5) configured for mounting the main body (1a) on the palm side of the wrist; and
a regulating portion (4) for restricting movement of the hand, said regulating portion being configured to extend along the direction of the hand and being configured to be placed on the palm side of the hand **characterized by** the said regulating portion comprising two separate parts (4a, 4b) integrally combined through a slide mechanism so that the length of the regulating portion is adjustable.

6. The vital sign detection device of claim 5, wherein the regulating portion (4) is also configured to be provided on the back side of the hand.

7. The vital sign detection device of claim 5, further comprising a second regulating portion (4b₁) configured to be provided on the back side of the hand.

8. The vital sign detection device of claim 7, further comprising a band (5) for tying the hand to the regulating portion (4).

9. The vital sign detection device of claim 7, further comprising a band (5) for tying the hand to the regulating portions (4b₁, 4b₂).

## Patentansprüche

1. Biosignal-Nachweisvorrichtung, welche für eine Anbringung an einem Handgelenk eines Subjekts eingerichtet ist, aufweisend:
einen zur Anbringung auf einer Handflächenseite des Handgelenks eingerichteten Grundkörper (101);
eine Druckabfühleinheit (220) zum Abfühlen einer Pulswelle;
eine Unterdrucksetzungseinheit zum Aufbringen von Druck auf einen Nachweisabschnitt des Handgelenks;
eine zur Anbringung des Grundkörpers an der Handflächenseite des Handgelenks eingerichtete Manschette (102); und
einen Regulierungsabschnitt zur Beschränkung der Bewegung der Hand, wobei der Regulierungsabschnitt so eingerichtet ist, dass er sich entlang der Richtung der Hand erstreckt, und so eingerichtet, dass er auf einer Handflächenseite der Hand angeordnet werden kann; **gekennzeichnet durch**
einen Bewegungsmechanismus (101a) zum Bewegen des Grundkörpers (101) oder eines Abschnitts des Grundkörpers zum distalen Ende der Hand hin, so dass der Grundkörper oder der Abschnitt des Grundkörpers als der Regulierungsabschnitt dient.

2. Biosignal-Nachweisvorrichtung nach Anspruch 1, welche ferner einen Schaltmechanismus aufweist, welcher die Vorrichtung aktiviert, wenn der Grundkörper (101) oder der Abschnitt des Grundkörpers zum distalen Ende der Hand hin bewegt wird.

3. Biosignal-Nachweisvorrichtung nach Anspruch 1, wobei der zum distalen Ende bewegte Abschnitt des Grundkörpers (101) ein zum distalen Ende hin erstreckbarer Führungsabschnitt (120) ist.

4. Biosignal-Nachweisvorrichtung nach Anspruch 3, wobei der Führungsabschnitt (120) lösbar am Grundkörper (101) angebracht ist.

5. Biosignal-Nachweisvorrichtung, welche für eine Anbringung an einem Handgelenk eines Subjekts eingerichtet ist, aufweisend:
einen zur Anbringung auf der Handflächenseite des Handgelenks aufgebauten Grundkörper (1a);
eine Druckabfühleinheit (2) zum Abfühlen einer Pulswelle;
eine Unterdrucksetzungseinheit zum Aufbringen von Druck auf die Druckabfühleinheit an einem Nachweisabschnitt des Handgelenks;
eine zur Anbringung des Grundkörpers (1a) an der Handflächenseite des Handgelenks eingerichtete Manschette (5); und
einen Regulierungsabschnitt (4) zur Beschränkung der Bewegung der Hand, wobei der Regulierungsabschnitt so eingerichtet ist, dass er sich entlang der Richtung der Hand erstreckt, und so eingerichtet, dass er auf einer Handflächenseite der Hand angeordnet werden kann, **dadurch gekennzeichnet, dass** der Regulierungsabschnitt zwei getrennte Teile (4a, 4b) aufweist, die über einen Gleitmechanismus zusammengefügt sind, so dass die Länge des Regulierungsabschnitts einstellbar ist.

6. Biosignal-Nachweisvorrichtung nach Anspruch 5, wobei der Regulierungsabschnitt (4) auch für ein Vorsehen auf der Rückseite der Hand eingerichtet ist.

7. Biosignal-Nachweisvorrichtung nach Anspruch 5, welche ferner einen zweiten Regulierungsabschnitt (4b₁) aufweist, der für ein Vorsehen auf der Rückseite der Hand eingerichtet ist.

8. Biosignal-Nachweisvorrichtung nach Anspruch 7, welche ferner ein Band (5) zum Binden der Hand an den Regulierungsabschnitt (4) aufweist.

9. Biosignal-Nachweisvorrichtung nach Anspruch 7, welche ferner ein Band (5) zum Binden der Hand an die Regulierungsabschnitte (4b₁, 4b₂) aufweist.

## Revendications

1. Dispositif de détection de signaux vitaux configuré pour être monté sur un poignet d'une main d'un sujet, comprenant :
■ un organe principal (101) configuré pour être monté sur un côté paume du poignet ;
■ une unité détectrice de pression (220) pour détecter une onde d'impulsion ;
■une unité de pressurisation pour appliquer une pression sur une partie de détection du poignet ;
■ une manchette (102) configurée pour le montage de l'organe principal sur le côté paume du poignet ; et
■ une partie régulatrice pour limiter le mouvement de la main, ladite partie régulatrice étant configurée pour s'étendre le long de la direction de la main et étant configurée pour être placée sur un côté paume de la main ; **caractérisée par**
■ un mécanisme mobile (101a) pour déplacer l'organe principal (101) ou une partie de l'organe principal vers l'extrémité distale de la main pour que l'organe principal ou la partie de l'organe principal serve de partie régulatrice.

2. Dispositif de détection de signaux vitaux selon la revendication 1, comprenant en outre un mécanisme de commutation qui active le dispositif quand l'organe principal (101) ou la partie de l'organe principal est déplacé(e) vers l'extrémité distale de la main.

3. Dispositif de détection de signaux vitaux selon la revendication 1, dans lequel la partie de l'organe principal (101) déplacée vers l'extrémité distale est une partie guide (120) extensible vers l'extrémité distale.

4. Dispositif de détection de signaux vitaux selon la revendication 3, dans lequel la partie guide (120) est montée de manière amovible sur l'organe principal (101).

5. Dispositif de détection de signaux vitaux configuré pour être monté sur un poignet d'une main d'un sujet, comprenant :
■ un organe principal (1a) configuré pour être monté sur un côté paume du poignet ;
■ une unité détectrice de pression (2) pour détecter une onde d'impulsion ;
■ une unité de pressurisation pour appliquer une pression sur l'unité détectrice de pression sur une partie de détection du poignet ;
■ une manchette (5) configurée pour monter l'organe principal (1a) sur le côté paume du poignet ; et
■ une partie régulatrice (4) pour limiter le mouvement de la main, ladite partie régulatrice étant configurée pour s'étendre le long de la direction de la main et étant configurée pour être placée sur le côté paume de la main,
**caractérisée en ce que** ladite partie régulatrice comprenant deux parties séparées (4a, 4b) intégralement combinées à travers un mécanisme coulissant de sorte que la longueur de la partie régulatrice soit réglable.

6. Dispositif de détection de signaux vitaux selon la revendication 5, dans lequel la partie régulatrice (4) est également configurée pour être fournie sur le côté dos de la main.

7. Dispositif de détection de signaux vitaux selon la revendication 5, comprenant en outre une seconde partie régulatrice (4bl) configurée pour être fournie sur le côté dos de la main.

8. Dispositif de détection de signaux vitaux selon la revendication 7, comprenant en outre une bande (5) pour lier la main à la partie régulatrice (4).

9. Dispositif de détection de signaux vitaux selon la revendication 7, comprenant en outre une bande (5) pour lier la main aux parties régulatrices (4b1, 4b2).
